# EUROPÄISCHE PATENTANMELDUNG

(11) **EP 4 613 238 A2**
(43) Veröffentlichungstag der Anmeldung: **10.09.2025**
(21) Anmeldenummer: 25192169.8
(22) Anmeldetag: 09.11.2017
(51) Int. Cl.: A61C 13/00

(54) **ANLAGE UND VERFAHREN FÜR DAS RECYCLING VERUNREINIGTER POLYOLEFINE**

(62) Teilanmeldung aus: 17800801.7
(71) Anmelder: Alpla-Werke Alwin Lehner GMBH & Co.KG, 6971 Hard (AT)
(72) Erfinder: Siegl, Robert, 6850 Dornbirn (AT); Weber, Andreas, 6712 Thüringen (AT)
(74) Vertreter: Swisspat Riederer Hasler Patentanwälte AG

(57) **Zusammenfassung**

Die Erfindung betrifft ein Recycling-Verfahren für verunreinigte Polyolefine, insbesondere HDPE, bei dem das verunreinigte Polyolefin mittels eines Lösungsmittels aufgequollen wird, wobei sich im Polyolefin vorhandene Verunreinigungen im Lösungsmittel lösen, und danach das Lösungsmittel und die im Lösungsmittel gelösten Verunreinigungen aus dem Polyolefin entfernt werden. Das verunreinigte Polyolefin-Material ist vor dem Aufquellen zu Stücken, zum Beispiel Flakes, verarbeitet worden. Die Stücke weisen eine maximale Abmessung von höchstens 40, 30 oder 25 Millimeter auf, wobei die maximale Abmessung durch den Abstand der am weitesten voneinander entfernten Punkte eines Stückes definiert ist.

## Beschreibung

### Technisches Gebiet

Die Erfindung betrifft neben einer Recycling-Anlage ein Recycling-Verfahren für verunreinigte Polyolefine, insbesondere für HDPE aus Verpackungsmaterial wie z.B. Getränkebehältern.

### Stand der Technik

Mit dem bevölkerungsbedingt steigenden Ressourcenverbrauch gewinnen Recycling-Verfahren insbesondere für im Verpackungsbereich zur Anwendung kommende Polymere an Bedeutung. Bei Lebensmittelverpackungen ist diese Wiederverwendung jedoch stark eingeschränkt, da die recycelten Materialien sich erneut für den direkten Lebensmittelkontakt eignen sollen, was voraussetzt, dass allfällige Verunreinigungen im Material sich nicht in unzulässigen Mengen auf die Lebensmittel übertragen. Die sich ständig verschärfenden gesetzlichen Anforderungen im Lebensmittelbereich limitieren den Einsatz von recyceltem Material und führen - abhängig von der Art des Polymers - speziell im Verpackungsbereich zu sehr schlechten Recycling-Raten. Während PET-Verpackungen in dieser Hinsicht relativ unproblematisch sind und das Material von PET-Flaschen (in begrenztem Umfang) wiederum für die Herstellung von PET-Flaschen verwendet werden werden kann, besteht bei Polyolefinen wie HDPE ein Nachholbedarf. Einerseits erschwert die Vielzahl unterschiedlicher HDPE-Verpackungsmaterialien das Recycling und andererseits ist die Entfernung von Verunreinigungen aus dem Material von HDPE-Flaschen ein noch nicht zufriedenstellend gelöstes Problem.

Die gemessene Reduktion einer angenommenen maximal möglichen Kontamination ("Challenge-Test"-Konzentration) auf eine Restkonzentration gilt als Mass für die Reinigungsleistung eines Recycling-Prozesses anhand dessen verschiedene Recycling-Prozesse bewertet und verglichen werden können. Manche der bisher bewerteten Verfahren sind durchaus geeignet, ein HDPE-Material aufzureinigen, benötigen dazu jedoch so lange, dass eine wirtschaftliche Nutzung nicht möglich ist.

### Aufgabe der Erfindung

Es ist eine Aufgabe der vorliegenden Erfindung, ein alternatives Recycling-Verfahren für verunreinigte Polyolefine bereitzustellen, wobei das durch das Verfahren erhaltene gereinigte Polyolefin-Material vorzugsweise für die Verwendung in Lebensmittelverpackungen geeignet ist. Darüber hinaus soll das Verfahren wirtschaftlich durchführbar sein. Weitere Vorteile der vorliegenden Erfindung ergeben sich aus der nachfolgenden Beschreibung.

### Darstellung der Erfindung

Die oben genannte Aufgabe wird gelöst durch ein Verfahren nach Anspruch 1 sowie eine Recycling-Anlage nach Anspruch 10.

Offenbart ist unter anderem ein Recycling-Verfahren für verunreinigte Polyolefine, wobei ein verunreinigtes Polyolefin-Material in der Gegenwart eines Lösungsmittels aufgequollen wird, wobei sich im Polyolefin-Material vorhandene Verunreinigungen im Lösungsmittel lösen, und das Lösungsmittel und die im Lösungsmittel gelösten Verunreinigungen aus dem Polyolefin-Material entfernt werden.

Offenbart ist zudem eine Recycling-Anlage für das Recycling von verunreinigten Polyolefinen mit einem Quellungs-Reaktor, beispielsweise in der Form eines Rührkessels oder einer Rührkesselkaskade oder eines Röhren-Reaktors, enthaltend ein Lösungsmittel, wobei es sich beim Lösungsmittel um ein Lösungsmittel mit geringerer Polarität als Wasser handelt, wobei der Quellungs-Reaktor dazu ausgebildet ist, ein verunreinigtes Polyolefin-Material in der Gegenwart des Lösungsmittels aufzuquellen, um im Polyolefin-Material vorhandene Verunreinigungen im Lösungsmittel zu lösen. Dem Quellungs-Reaktor nachgeschaltet (oder als Teil des Quellungs-Reaktors) enthält die Recycling-Anlage einen Anlagenteil zum Entfernen des Lösungsmittels und der im Lösungsmittel gelösten Verunreinigungen aus dem Polyolefin-Material, zwecks Erhalt eines gereinigten Polyolefin-Materials.

Nachfolgend werden Merkmale beschrieben, wobei diese (individuell) als bevorzugte Merkmale zu betrachten sind, auch wenn sie nicht explizit als solche bezeichnet werden. Die Merkmale seien separat (als Teil eines beliebigen Verfahrens und/oder einer beliebigen Anlage) und - soweit sie sich nicht ausschliessen - in beliebiger Kombination offenbart. Dies schliesst die Möglichkeit der gleichzeitigen Verwirklichung aller beschriebenen Merkmale ein.

Von besonderem Interesse als Polyolefin, welches mit Hilfe des in diesem Dokument gelehrten Verfahren und/oder mit der in diesem Dokument gelehrten Recycling-Anlage gereinigt werden kann, ist HDPE ("high-density polyethylene"). Deshalb wird dieses Polymer im Folgenden beispielhaft zur Erklärung der Erfindung verwendet. Das zu HDPE Offenbarte sei jedoch alternativ auch für Polyolefine im Allgemeinen und insbesondere die in diesem Dokument genannten bevorzugten Polyolefine offenbart.

Verunreinigungen, insbesondere solche aus unpolaren (apolaren) Molekülen, können in die HDPE Matrix hinein migrieren und sich dort in sehr hohen Konzentrationen ansammeln. Sie werden von der HDPE Matrix nur sehr langsam wieder freigegeben, auch wenn hohe Temperaturen (100-300°C) und ein hohes Konzentrationsgefälle zur Entfernung dieser Verunreinigungen im Recycling-Prozess eingesetzt werden.

Mit der vorliegenden Erfindung soll das HDPE so verändert werden, dass es Verunreinigungen leichter und mit geringerem technischen Aufwand wieder freigibt. Dazu wird die Quellbarkeit von Polyolefinen ausgenutzt: Ein aufgequollenes Polymer ist in der Regel voluminöser, wobei die Molekülabstände grösser und die Dichte kleiner sind. Es lässt Verunreinigungen deshalb viel schneller nach aussen diffundieren als ein nicht aufgequollenes Polymer bei ansonsten gleichem Konzentrationsgefälle der Verunreinigungen. Darüber hinaus können zwei zusätzliche Effekte die Reinigungswirkung deutlich verstärken. Einerseits lässt sich ein aufgequollenes Polymer mechanisch wie ein Schwamm auspressen, wodurch viele der darin vorhandenen Verunreinigungen mit entfernt werden. Andererseits werden beim Entfernen des für das Quellen verwendeten Lösungsmittels Verunreinigungen mitgerissen.

Es ist deshalb ein Recycling-Verfahren für verunreinigte Polyolefine offenbart, welches in einer Recycling-Anlage wie zum Beispiel der in diesem Dokument beschriebenen Anlage durchgeführt werden kann.

Das Recycling-Verfahren beinhaltet die Entfernung von Verunreinigungen aus einem verunreinigten Polyolefin-Material, insbesondere aus grenzflächenfernen Bereichen des verunreinigten Polyolefin-Materials.

Die Entfernung von Verunreinigungen aus dem verunreinigten Polyolefin-Material beinhaltet, dass das verunreinigte Polyolefin-Material in der Gegenwart eines Lösungsmittels und/oder durch ein Lösungsmittel aufgequollen wird. Dabei können sich im Polyolefin-Material vorhandene Verunreinigungen im Lösungsmittel, das sich im Polyolefin-Material befindet, lösen. Das Lösungsmittel und die im Lösungsmittel gelösten Verunreinigungen können danach zusammen und/oder gleichzeitig aus dem Polyolefin-Material entfernt werden.

Das Recycling-Verfahren kann hierbei alle Kriterien der European Food Safety Authority (EFSA) entsprechend der EU Regulation EC282/2008 und der EU Regulation 1935/2004 sowie deren Ergänzungen erfüllen.

Eine Bewertung der Recyclingleistung des Recycling-Verfahrens kann hierbei durch das CEF Panel (EFSA Panel on Food Contact Materials, Enzymes, Flavourings and Processing Aids) anhand eine sogenannten Challenge Tests erfolgen, in dem das Polyolefin-Material mit vorbestimmten Kontaminationen in vorbestimmten Konzentrationen verunreinigt wird und die Konzentrationen der Kontaminationen nach Durchführung des Recycling-Verfahrens gemessen wird. Durch das Recycling-Verfahren können allgemeine Verunreinigungen durch Benzol, Toluol und/oder Xylol in ihrer Konzentration stark reduziert oder sogar komplett entfernt werden. Durch das Recycling-Verfahren können spezielle Verunreinigungen wie Di-tert-butylhydroxyltoluol (BHT), Phenylcyclohexan, Methylstearat, Zinkstearat, Trichlorethan, Trichlormethan, Butylsalicylat, Methylpalmitat, Lindan und/oder Benzophenon in ihrer Konzentration stark reduziert oder sogar komplett entfernt werden. Natürlich können auch jene Kontaminationen in ihrer Konzentration stark reduziert oder sogar komplett entfernt werden, die sich in den verwendeten Lösungsmittel befinden können.

Durch das Recycling-Verfahren kann gereinigtes Polyolefin-Material bereitgestellt werden, welches zumindest teilweise für Lebensmittelkontakt geeignet ist. Damit kann des gereinigte Polyolefin-Material für Verpackungen verwendet werden, die zum Verpacken von Lebensmittel geeignet sind.

Das verunreinigte Polyolefin-Material kann aus einem oder mehreren Polyolefinen hergestellt sein und/oder einen Anteil an Polyolefinen von gesamthaft mindestens 60, 80 oder 90 Gewichtsprozent enthalten.

Beim einen oder den mehreren Polyolefinen kann es sich mit Vorteil um PP (Polypropylen) und/oder PE (Polyethylen) handeln, insbesondere um LDPE (Low Density Polyethylene) und/oder LLDPE (Linear Low Density Polyethylene) und/oder HDPE (High Density Polyethylene) und/oder UHMWPE (Ultra High Molecular Weight Polyethylene), wobei HDPE bevorzugt ist.

Alternativ oder zusätzlich kann es sich beim einen oder den mehreren Polyolefinen um ein thermoplastisches Elastomer oder ein Plastomer handeln.

Vorzugsweise enthält das Polyolefin-Material weniger als 20 oder 10 Gewichtsprozent Fremdpolymere wie zum Beispiel Barriereschichten.

Das verunreinigte Polyolefin-Material liegt zweckmässigerweise in fester Form vor, insbesondere beim Aufquellen.

Das verunreinigte Polyolefin-Material kann vor dem Aufquellen zu Stücken (zum Beispiel "Flakes") verarbeitet worden sein und/oder es liegt beim Aufquellen bereits in der Form von Stücken vor, wobei die Stücke vorzugsweise eine maximale Abmessung (Abstand der am weitesten voneinander entfernten Punkte) von höchstens 40, 30 oder 25 Millimeter und/oder mindestens 0.25, 0.5 oder 1 Millimeter aufweisen. Dies erleichtert das Aufquellen und wegen der grossen Oberfläche den Stoffaustausch und damit die Reinigung. Vorzugsweise besitzen mindestens 90 oder 95 Gewichtsprozent des verunreinigten Polyolefin-Materials diese Form.

Das verunreinigte Polyolefin-Material kann vor dem Aufquellen von Fremdstoffen wie Leim oder Papier getrennt und gegebenenfalls nach Farben sortiert werden.

Das verunreinigte Polyolefin-Material stammt mit Vorteil aus Lebensmittelverpackungen, insbesondere Flaschen.

Nach einer Variante handelt es sich bei den im verunreinigten Polyolefin-Material vorhandenen Verunreinigungen um sogenannte "NIAS" ("nonintentionally added substances").

Die im verunreinigten Polyolefin-Material vorhandenen Verunreinigungen können (insbesondere in der Mehrzahl und/oder in einem Anteil von zusammen mindestens 40, 60 oder 80 Gewichtsprozent der Verunreinigungen) Moleküle umfassen, die unpolar oder im Wesentlichen unpolar sind und/oder mindestens 5, 8 oder 10 und/oder höchstens 70, 50 oder 40 Kohlenstoffatome aufweisen und/oder ein Molekulargewicht von mindestens 20 Dalton und höchstens 1000 Dalton aufweisen.

Alternativ oder zusätzlich können die im verunreinigten Polyolefin-Material vorhandenen Verunreinigungen (insbesondere in der Mehrzahl und/oder in einem Anteil von zusammen mindestens 40, 60 oder 80 Gewichtsprozent der Verunreinigungen) Stoffe aus einer oder mehreren der folgenden Stoffgruppen umfassen: Aliphatische gesättigte Kohlenwasserstoffe, aliphatische ungesättigte Kohlenwasserstoffe, aliphatische Kohlenwasserstoffe mit funktionellen Gruppen, aromatische Kohlenwasserstoffe, aromatische Kohlenwasserstoffe mit funktionellen Gruppen, Duftstoffe (insbesondere Terpene wie Limonen), Geschmacksstoffe.

Die im verunreinigten Polyolefin-Material vorhandenen Verunreinigungen können (insbesondere in der Mehrzahl und/oder in einem Anteil von zusammen mindestens 40, 60 oder 80 Gewichtsprozent der Verunreinigungen) Moleküle umfassen, die unter den Begriff MOSH (Mineral Oil Saturated Hydrocarbons) und/oder unter den Begriff MOAH (Mineral Oil Aromatic Hydrocarbons) fallen.

Durch das Aufquellen des verunreinigten Polyolefin-Materials in der Gegenwart des Lösungsmittels wird ein aufgequollenes Polyolefin-Material erhalten.

Das Aufquellen wird vorzugsweise durchgeführt, bis das Volumen des verunreinigten Polyolefin-Materials um mindestens 0.3, 0.5 oder 0.8 und/oder höchstens 40, 20 oder 7 Prozent zugenommen hat, wobei eine Volumenzunahme um 1 bis 5 Prozent besonders bevorzugt ist.

Vorzugsweise wird das verunreinigte Polyolefin-Material bei einer Temperatur von mindestens 0, 100 oder 150 Grad Celsius und/oder höchstens etwa 10°C unterhalb der Schmelztemperatur des jeweiligen Polyolefins aufgequollen.

Weiter ist es von Vorteil, wenn das verunreinigte Polyolefin-Material bei Überdruck, insbesondere bei einem Druck von mindestens 1, 50 oder 100 bar und/oder höchstens 1000, 500 oder 250 bar aufgequollen wird.

Alternativ oder zusätzlich kann vorgesehen sein, dass das verunreinigte Polyolefin-Material in der Gegenwart eines Lösungsmittels aufgequollen wird, wobei das Lösungsmittel in flüssigem Zustand oder im superkritischen Zustand vorliegt.

Das verunreinigte Polyolefin-Material kann zum Beispiel während mindestens 0.5, 1 oder 2 Stunden und/oder höchstens 10, 5 oder 3 Stunden aufgequollen werden.

Es ist von Vorteil, wenn das Polyolefin-Material und/oder das Lösungsmittel während des Aufquellens (insbesondere relativ zueinander) bewegt werden. Beispielsweise können das Polyolefin-Material und/oder das Lösungsmittel gerührt werden.

Gemäss einer Variante wird das Aufquellen des verunreinigten Polyolefin-Materials mittels eines Perkolationsverfahrens (Durchlaufverfahren) durchgeführt. Dabei strömt das Lösungsmittel am verunreinigten Polyolefin-Material vorbei und das verunreinigte Polyolefin-Material saugt das Lösungsmittel auf. Durch den Fluss des Lösungsmittels am Polyolefin-Material vorbei ist dafür gesorgt, dass lokal mit Verunreinigungen beladene Lösungsmittel abfließt und durch weniger beladenes ersetzt wird.

Nach einer weiteren Variante wird das Aufquellen des verunreinigten Polyolefin-Materials mittels eines Immersionsverfahrens durchgeführt. Dabei wird das verunreinigte Polyolefin-Material in das Lösungsmittel eingetaucht, wobei Polyolefin-Material und Lösungsmittel vorzugsweise gerührt werden. Dadurch wird ein Ausgleich der Konzentration der Verunreinigungen im Lösungsmittel erleichtert.

Nach einer weiteren Variante wird das Aufquellen des verunreinigten Polyolefin-Materials durchgeführt, während das Polyolefin-Material und das Lösungsmittel im Gegenstrom geführt werden. Vorzugsweise weist das Lösungsmittel zu jeder Zeit des Stoffaustausches eine geringere Konzentration der Verunreinigungen auf, als das verunreinigte Polyolefin-Material mit dem es gerade in Kontakt ist, wodurch der Konzentrationsgradient immer von der Polyolefinphase in Richtung der Lösungsmittelphase abfällt. Hierzu wird nicht oder nur geringfügig verunreinigtes Lösungsmittel dort eingespeist, wo das "ausgelaugte" verunreinigte Polyolefin-Material abgezogen wird. Vice versa wird dort, wo das verunreinigte Polyolefin-Material zugeführt wird, das nun mit den Verunreinigungen beladene Lösungsmittel abgezogen. Dies geschieht in den dem Fachmann bekannten verfahrenstechnischen Apparaturen, bevorzugt Rohrreaktoren oder Rührkesselkaskaden.

Das Lösungsmittel mit den darin gelösten Verunreinigungen wird in diesem Dokument auch als "verunreinigtes Lösungsmittel" bezeichnet. Das verunreinigte Lösungsmittel kann gereinigt und wiederum als Lösungsmittel im Verfahren, insbesondere zum Aufquellen des verunreinigten Polyolefin-Materials, verwendet werden, wobei die Reinigung des verunreinigten Lösungsmittels beispielsweise mittels einer Destillation oder Rektifikation erfolgen kann.

Beim Lösungsmittel kann es sich um ein Lösungsmittel geringer Polarität oder um ein unpolares Lösungsmittel handeln. Einerseits kann das Lösungsmittel vorzugsweise weniger polar als Wasser, bevorzugt gleich oder weniger polar als Aceton sein. Andererseits kann das Lösungsmittel gleich oder polarer als n-Hexan sein.

Gemäss einer Variante handelt es sich beim Lösungsmittel um ein Alkan, insbesondere n-Hexan oder n-Heptan.

Bevorzugt weisen das Lösungsmittel und das verunreinigte Polyolefin-Material eine RED ("relative energy difference") nach Hansen (Quelle: Charles M. Hansen: Hansen Solubility Parameters: A User's Handbook, 2nd edition, CRC Press, Boca Raton, 2007, ISBN 0-8493-7248-8) von höchstens 3, 2, oder 1 auf.

Das für das vorliegende Verfahren verwendete Lösungsmittel kann aus einer einzigen Art von Lösungsmittel oder aus mehreren Arten von Lösungsmitteln, d.h. aus einem Lösungsmittelgemisch, bestehen. Vorzugsweise besteht das Lösungsmittel zu mindestens 80, 90, oder 95 Gewichtsprozent aus einer Art von Lösungsmittel, beispielsweise aus n-Hexan, da es so leichter zu reinigen ist.

Wenn das Lösungsmittel mehr als eine Art von Lösungsmittel enthält, so ist es bevorzugt, dass die Siedepunkte von zwei der verwendeten Arten von Lösungsmitteln sich um mindestens 2, 5 oder 10 Grad Celsius und/oder höchstens 100, 50 oder 30 Grad Celsius unterscheiden, damit sie einfach mittels Destillation getrennt werden können. Weiter ist bevorzugt wenn die Lösungsmittel kein Azeotrop bilden.

Die Entfernung des Lösungsmittels und/oder der im Lösungsmittel gelösten Verunreinigungen aus dem Polyolefin-Material kann zweckmässigerweise eine mechanische Kompression des aufgequollenen Polyolefin-Materials beinhalten, wodurch ein ausgepresstes Polyolefin-Material erhalten wird. Für die mechanische Kompression des aufgequollenen Polyolefin-Materials kann zum Beispiel eine Schnecke oder eine Bandfilterpresse verwendet werden.
Durch die mechanische Kompression können mit Vorteil mindestens 60, 80 oder 90 Prozent des Lösungsmittels und/oder der im Lösungsmittel gelösten Verunreinigungen aus dem Polyolefin-Material entfernt werden.

Das aufgequollene Polyolefin-Material weist vorzugsweise ein um mindestens 0.3, 0.5 oder 0.8 und/oder höchstens 40, 20 oder 7 Prozent grösseres Volumen auf als das nicht aufgeqollene Polyolefin-Material und/oder als das ausgepresste Polyolefin-Material.

Gemäss einer Variante können der Verfahrensschritt, bei dem das Polyolefin-Material gequollen wird und der Verfahrensschritt, bei dem das gequollene Polyolefin-Material einer mechanischen Kompression unterworfen wird, jeweils ein Mal durchgeführt werden. Alternativ können die genannten Verfahrensschritte mehrmals (zum Beispiel jeweils zwei, drei oder mehr Male) während des Verfahrens und/oder mit demselben Polyolefin-Material durchgeführt werden.

Es kann also z.B. vorgesehen sein, dass das aufgequollene Polyolefin-Material einer mechanischen Kompression unterworfen wird, wobei ein Teil (insbesondere mindestens 30, 20 oder 10 Prozent) des im aufgequollenen Polyolefin-Material vorhandenen (verunreinigten) Lösungsmittels aus dem Polyolefin-Material entfernt wird. Anschliessend kann das erhaltene ausgepresste Polyolefin-Material in der Gegenwart eines (d.h. desselben oder eines anderen oder eines gereinigten) Lösungsmittels wiederum aufgequollen werden, wobei sich im Polyolefin-Material vorhandene (verbliebene) Verunreinigungen im Lösungsmittel lösen. Danach kann das erhaltene aufgequollene Polyolefin-Material erneut einer mechanischen Kompression unterworfen werden. Gemäss einer Variante kann das Lösungsmittel nach einer oder jeder mechanischen Kompression gereinigt werden, bevor es erneut zum Aufquellen verwendet wird. Nach einer anderen Variante kann diese Reinigung unterbleiben.

Das Recycling-Verfahren und/oder die Entfernung des Lösungsmittels (vorzugsweise mit im Lösungsmittel gelösten Verunreinigungen) aus dem Polyolefin-Material kann eine (erste) Trocknung des Polyolefin-Materials (insbesondere des ausgepressten Polyolefin-Materials) in einem Trockner umfassen, wobei im Polyolefin-Material verbliebenes Lösungsmittel verdampft wird, wodurch ein getrocknetes Polyolefin-Material erhalten wird.

Obwohl dies nicht die erste Trocknung des Polyolefin-Materials sein muss, wird diese Trocknung als "(erste) Trocknung" bezeichnet, (lediglich) um sie von der weiter unten beschriebenen "zweiten Trocknung" zu unterscheiden.

Mit Vorteil wird das Polyolefin-Material zur (ersten) Trocknung mit einem Gas, insbesondere einem Inertgas wie zum Beispiel Stickstoff, beaufschlagt. Das verdampfte Lösungsmittel kann z.B. mittels einer Kältefalle aus dem Gas zurückgewonnen werden.

Es ist auch denkbar, dass die (erste) Trocknung unter Vakuum und/oder in einem Vakuum-Trockner durchgeführt wird und/oder dass sie bei Unterdruck, insbesondere bei einem Druck von unter 1 (Feinvakuum), 300 (Grobvakuum) oder 1013 (Unterdruck) mbar absolut durchgeführt wird.

Die (erste) Trocknung wird zweckmässigerweise bei einer Temperatur durchgeführt, die (vorzugsweise mindestens 5 oder 10 Grad Celsius und/oder höchstens 50, 30 oder 20 Grad Celsius) unterhalb der Schmelztemperatur (Peaktemperatur nach ISO11357-3-2013) des Polyolefin-Materials liegt und/oder das Gas, mit dem das Polyolefin-Material zur (ersten) Trocknung beaufschlagt wird, besitzt eine solche Temperatur.

Sinnvollerweise wird die (erste) Trocknung bei einer Temperatur durchgeführt, die über der Siedetemperatur des Lösungsmittels und/oder der im Polyolefin-Material verbliebenen Verunreinigungen liegt.

Die (erste) Trocknung wird vorzugsweise bei einem Druck von mindestens 1, 300 oder 1013 mbar absolut und/oder höchstens dem Dampfdruck des Lösungsmittels bei der entsprechenden Trocknungstemperatur durchgeführt.

Die (erste) Trocknung wird vorzugsweise während einer Dauer von mindestens 60, 30 oder 10 Minuten und/oder höchstens 30, 20, oder 10 Stunden durchgeführt.

Beim zur (ersten) Trockung des Polyolefin-Materials verwendeten Trockner kann es sich zum Beispiel um einen Trommel-Trockner oder einem WirbelschichtTrockner handeln.

Das Recycling-Verfahren und/oder die Entfernung der Verunreinigungen aus dem verunreinigten Polyolefin-Material kann ein Schmelzen und Extrudieren des Polyolefin-Materials (insbesondere des getrockneten Polyolefin-Materials) umfassen. Anschliessend kann das Polyolefin granuliert werden, wodurch ein Polyolefin-Granulat erhalten wird.

Für die Extrusion wird vorzugsweise ein Extruder mit Entgasungszone eingesetzt. Dadurch kann im Polyolefin-Material verbliebenes Lösungsmittel entfernt werden.

Ausserdem kann vorgesehen sein, dass das Polyolefin-Material einer Schmelzefiltration unterzogen wird.

Bei der Entfernung des Lösungsmittels und/oder der Verunreinigungen aus dem Polymer-Material können auch nützliche Zusätze aus dem Polymer-Material entfernt werden. Es kann deshalb vorgesehen sein, dass dem Polyolefin-Material in geschmolzenem Zustand ein oder mehrere Zusätze (insbesondere im Wesentlichen dieselben Zusätze, die zuvor entfernt wurden) zugesetzt werden. Bei den ein oder mehreren Zusätzen kann es sich zum Beispiel um einen oder mehrere Prozessstabilisatoren wie Antioxidantien handeln.

Das Recycling-Verfahren und/ oder die Entfernung der Verunreinigungen aus dem verunreinigten Polyolefin-Material kann eine (zweite) Trocknung, insbesondere eine Vakuumtrocknung, des Polyolefin-Materials (insbesondere des Polyolefin-Granulats) umfassen. Die Trocknung kann bespielweise in einem Festphasenpolykondensationsreaktor ("Solid-State-Polycondensation-" bzw. "SSP-Reaktor"), durchgeführt werden.

Obwohl dies nicht die zweite Trocknung des Polyolefin-Materials sein muss, wird diese Trocknung als "(zweite) Trocknung" bezeichnet, (lediglich) um sie von der weiter oben beschriebenen "ersten Trocknung" zu unterscheiden.

Bevorzugt wird die (zweite) Trocknung bei Unterdruck (insbesondere bei einem Druck von unter 1013mbar (Atmosphärendruck bei Normbedingungen)) und/oder unter Vakuum unter 50mbar durchgeführt. Alternativ kann die (zweite) Trocknung bei Überdruck, insbesondere bei einem Druck bis zum Dampfdruck des jeweiligen Lösungsmittels bei der entsprechenden Trocknungstemperatur, durchgeführt werden und/ oder das Polyolefin-Material kann zur Trocknung mit einem Gas, insbesondere einem Inertgas wie zum Beispiel Stickstoff, beaufschlagt werden.

Die (zweite) Trocknung wird zweckmässigerweise bei einer Temperatur durchgeführt, die (vorzugsweise mindestens 5 oder 10 Grad Celsius und/oder höchstens 50, 30 oder 20 Grad Celsius) unterhalb der Schmelztemperatur (Peaktemperatur nach ISO11357-3-2013) des Polyolefin-Materials liegt und/ oder das Gas, mit dem das Polyolefin-Material zur Trocknung optional beaufschlagt wird, besitzt eine solche Temperatur.

Die (zweite) Trocknung wird vorzugsweise bei einem Druck von mindestens 1mbar, 5mbar oder 50mbar und/oder höchstens 1bar, 10bar oder 100bar durchgeführt.

Die (zweite) Trocknung wird vorzugsweise während einer Dauer von mindestens 1min, 5min oder 10minutenund/oder höchstens 30Stunden, 20Stunden, oder 10 Stunden durchgeführt.

Ferner kann die Trocknung in einer Atmosphäre erfolgen, die zu mindestens 99,98, 98, oder 90 Volumenprozent aus Stickstoff besteht.

Offenbart ist zudem eine Recycling-Anlage für das Recycling von verunreinigten Polyolefinen, insbesondere zur Durchführung des in diesem Dokument offenbarten Recycling-Verfahren und/ oder zur Entfernung von Verunreinigungen aus einem verunreinigten Polyolefin-Material. Beim Polyolefin-Material kann es sich insbesondere um das oben im Zusammenhang mit dem Recycling-Verfahren beschriebene Polyolefin-Material handeln.

Nach einer Variante kann es sich bei der Recycling-Anlage um eine aufgerüstete oder umgerüstete PET-Recycling-Anlage, d.h. eine Anlage zum Recycling von Polyethylenterephthalat handeln.

Die Recycling-Anlage weist einen Quellungs-Reaktor auf, beispielsweise in der Form eines Rührkessels oder einer Kaskade von Rührkesseln oder eines Röhren-Reaktors. Der Quellungs-Reaktor enthält ein Lösungsmittel, wobei es sich beim Lösungsmittel um ein Lösungsmittel mit geringerer Polarität als Wasser und/oder um das oben im Zusammenhang mit dem Recycling-Verfahren beschriebenes Lösungsmittel handelt. Der Quellungs-Reaktor ist dazu ausgebildet, das verunreinigte Polyolefin-Material in der Gegenwart des Lösungsmittels aufzuquellen, um im Polyolefin-Material vorhandene Verunreinigungen im Lösungsmittel zu lösen oder auszuwaschen.

Der Quellungs-Reaktor kann dazu ausgebildet sein, das verunreinigte Polyolefin-Material bei oder während der weiter oben im Zusammenhang mit dem Verfahren beschriebenen Temperatur und/oder Druck und/oder Zeit in der Gegenwart des Lösungsmittels aufzuquellen. Insbesondere kann der Quellungs-Reaktor dazu ausgebildet sein, das verunreinigte Polyolefin-Material bei einer Temperatur etwa 10°C unterhalb des Schmelzpunktes des jeweiligen Polyolefinmaterials und/oder bei einem Druck zwischen 1 und 1000 bar in der Gegenwart des Lösungsmittels aufzuquellen.

Weiter ist der Quellungs-Reaktor vorzugsweise dazu ausgebildet, das Polyolefin-Material während des Aufquellens zu bewegen, insbesondere durch Rühren. Zu diesem Zweck kann der Quellungs-Reaktor beispielsweise ein Rührwerk aufweisen.

Es kann vorgesehen sein, dass der Quellungs-Reaktor dazu ausgebildet ist, das Aufquellen des Polyolefin-Materials mittels eines Perkolationsverfahrens, eines Immersionsverfahrens oder dadurch zu bewerkstelligen, dass das Polyolefin-Material und das Lösungsmittel im Gegenstrom zueinander geführt werden, insbesondere wie dies oben im Zusammenhang mit dem Recycling-Verfahren beschrieben wurde.

Die Recycling-Anlage weist einen dem Quellungs-Reaktor nachgeschalteten (d.h. stromabwärts davon angeordneten) Anlagenteil zum Entfernen des Lösungsmittels und der im Lösungsmittel gelösten Verunreinigungen aus dem Polyolefin-Material auf.

Insbesondere kann ein solcher Anlagenteil einen Filter zum Abfiltern des Lösungsmittels und/oder Mittel zum Auspressen des aufgequollenen Polyolefin-Materials aufweisen.

Zweckmässigerweise besitzt die Recycling-Anlage eine oder mehrere dem Quellungs-Reaktor vorgeschaltete (d.h. stromaufwärts davor angeordnete) Trenn-Stufen und/oder Wasch-Stufen.

Diese können zum Abtrennen von Fremdmaterialien, wie zum Beispiel Papieretiketten, vom Polyolefin-Material ausgebildet sein.

Die Recycling-Anlage kann mit Vorteil eine oder mehrere der folgenden Trenn-Stufen aufweisen (insbesondere dem Quell-Reaktor in der angegebenen Reihenfolge vorgeschaltet): Metallabscheider (zur Abscheidung von magnetischen und/oder nicht magnetischen Metallen); Label-Remover (zur Entfernung von Etiketten); Ballistik-Sorter (zur Entfernung von Folien); Flaschen-Sorter (zur Sortierung nach Farbe); manuelle Sortier-Stufe; Windsichter (zum Beispiel zur Entfernung von Etiketten und Sleeves); Schwimm-Sink-Trenn-Stufe (Trennung durch Absinken im Wasser); Sortier-Stufe mittels NIR-Spektroskopie (Nahinfrarotspektroskopie; zur Abtrennung von Fremdkunststoffen und/oder Staub).

Die Recycling-Anlage kann mit Vorteil eine oder mehrere der folgenden Wasch-Stufen aufweisen (insbesondere dem Quell-Reaktor in der angegebenen Reihenfolge vorgeschaltet): Wasch-Stufe mit Wasser und gegebenenfalls einer oder mehreren waschaktiven Substanzen wie zum Beispiel einer Lauge; Wasch-Stufe mit heissem Wasser (zum Beispiel zur Entfernung von Papier und Karton); Wasch-Stufe zur Nachwäsche mit Wasser und/oder zur Neutralisation der Lauge.

Nach dem Trennen und/oder Waschen kann das Polymer-Material getrocknet werden, bevor es dem Quellungs-Reaktor zugeführt wird. Vorzugsweise ist also zwischen den dem Quell-Reaktor vorgeschalteten Trenn- und/oder Waschstufen und dem Quell-Reaktor ein Trockner angeordnet.

Die Recycling-Anlage kann eine Mühle zur Zerkleinerung von aus dem Polyolefin-Material hergestellten Produkten wie zum Beispiel Behältern und Verschlüssen aufweisen. Bevorzugte Stückgrössen sind oben im Zusammenhang mit dem Verfahren angegeben, wobei eine Verarbeitung zu Flakes besonders bevorzugt ist.

Die Mühle kann zum Beispiel einer oder mehrerer der oben genannten Trenn-Stufen nachgeschaltet und/oder einer oder mehreren der oben genannten Wasch-Stufen vorgeschaltet sein. Insbesondere kann das Waschen des Polymer-Materials nach der Zerkleinerung stattfinden.

Weiter kann die Recycling-Anlage einen dem Quellungs-Reaktor nachgeschalteten Trockner aufweisen, wie er zum Beispiel oben im Zusammenhang mit der (ersten) Trocknung beschrieben wurde.

Ein dem Quellungs-Reaktor nachgeschalteter Anlagenteil zur Reinigung des Lösungsmittels insbesondere mittels Destillation oder Rektifikation und zur Rückführung des gereinigten Lösungsmittels in den Quellungs-Reaktor ist vorzugsweise ebenfalls vorgesehen.

Schliesslich sei ein gereinigtes Polyolefin-Material offenbart, hergestellt durch ein in diesem Dokument beschriebenes Verfahren und/oder mittels einer in diesem Dokument beschriebenen Recycling-Anlage, wobei das gereinigte Polyolefin-Material vorzugsweise in der Form eines Granulats vorliegt.

Begriffe in diesem Dokument sollen bevorzugt so verstanden werden, wie sie ein Fachmann auf dem Gebiet verstehen würde. Sind im jeweiligen Kontext mehrere Interpretationen möglich, so sei vorzugsweise jede Interpretation individuell offenbart. Insbesondere für den Fall, dass Unklarheiten bestehen sollten, können alternativ oder ergänzend die in diesem Dokument aufgeführten bevorzugten Definitionen herangezogen werden.

Wenn in diesem Dokument von der Entfernung der im Lösungsmittel gelösten Verunreinigungen aus dem Polyolefin die Rede ist, so sei alternativ jeweils eine Entfernung von im Polyolefin vorhandenen Verunreinigungen offenbart. Dies deshalb, weil Reinigungsschritte wie z.B. die SSP nicht nur im Lösungsmittel gelöste Verunreinigungen aus dem Polyolefin zu entfernen vermögen, sondern auch solche, die nicht im Lösungsmittel gelöst sind.

Zudem seien die nachfolgenden Patentansprüche zusätzlich jeweils mit einem Rückbezug auf jeden beliebigen der vorhergehenden Patentansprüche ("nach einem der vorhergehenden Ansprüche") offenbart, auch wenn sie nicht in dieser Form beansprucht sind.

### Kurze Beschreibung der Zeichnungen

Es zeigen:

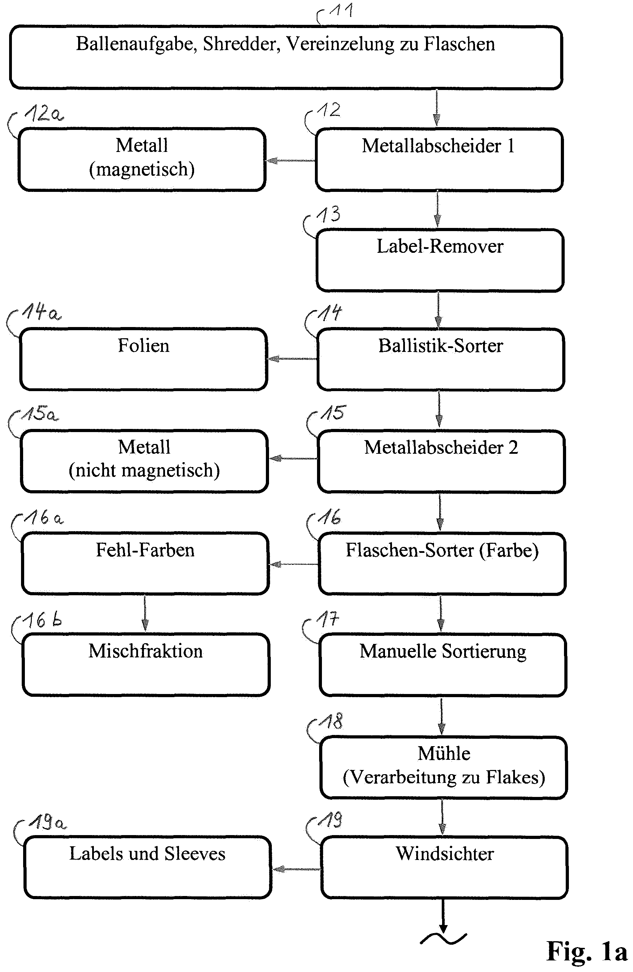

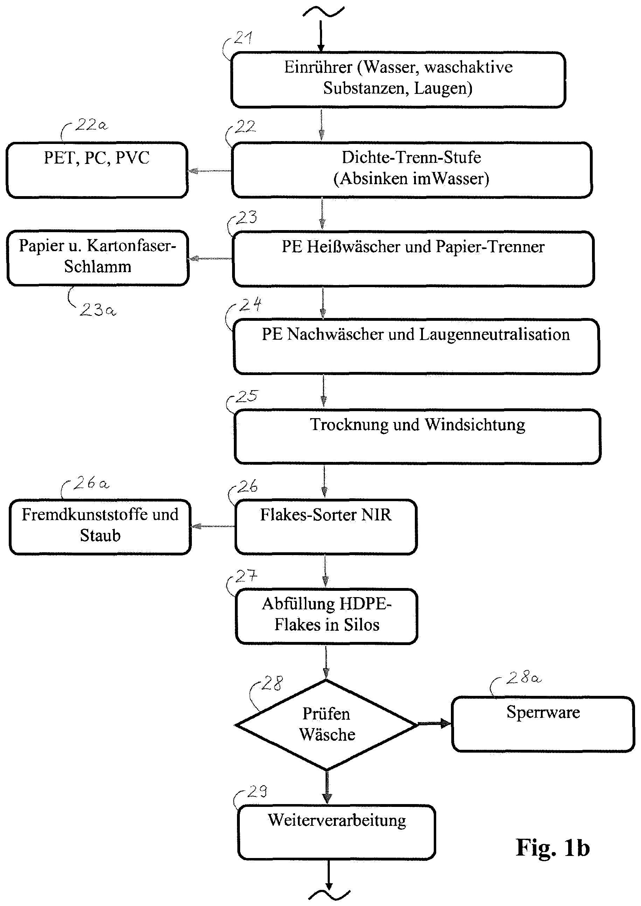

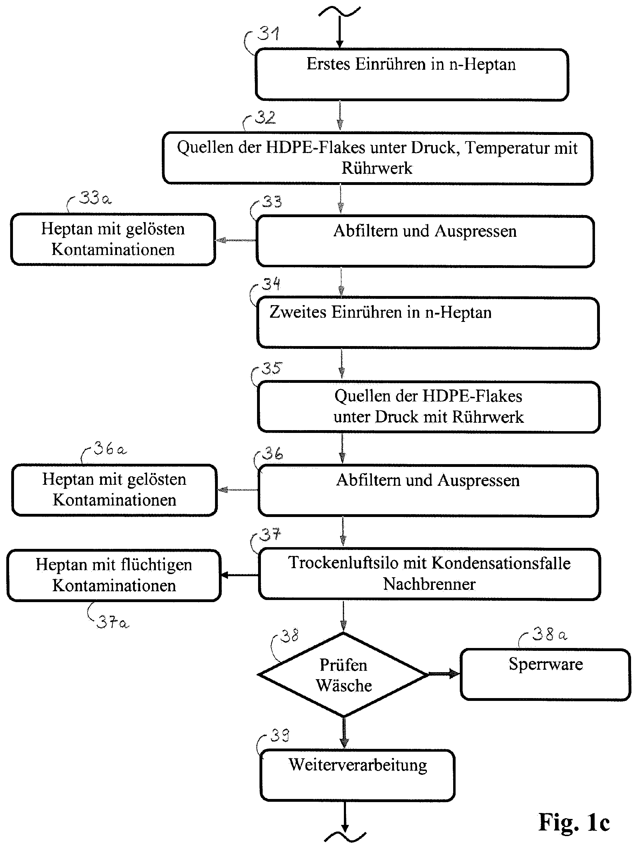

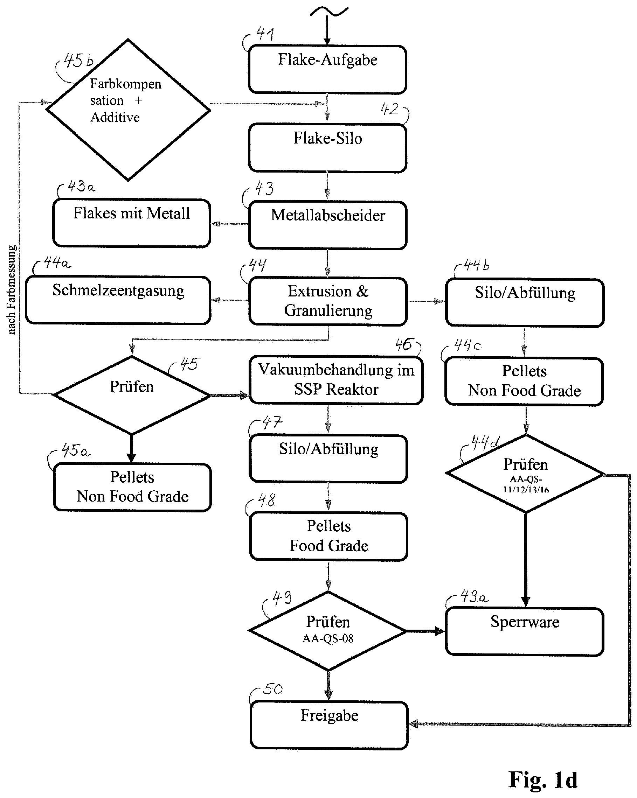

Fig. 1 a-d ein Recycling-Verfahren (Flussdiagramm in 4 Teilen in der Reihenfolge 1a, 1b, 1c, 1d)

### Ausführung der Erfindung

Die Erfindung wird nachfolgend anhand der Zeichnungen beispielhaft erläutert.

In den Fig. 1a bis 1d ist der Ablauf eines Recycling-Verfahrens für verunreinigte Polyolefine dargestellt. Das Verfahren besteht im Wesentlichen aus 4 Teilen:
In einem ersten Teil (dargestellt in Fig.1a) wird das Polyolefin-Material sortiert und anschliessend zerkleinert.

In einem zweiten Teil (dargestellt in Fig.1b) wird das zerkleinerte Polyolefin-Material gewaschen, getrocknet und gegebenenfalls nochmals sortiert.

In einem dritten Teil (dargestellt in Fig.1c) werden Verunreinigungen aus dem gewaschenen und getrockneten Polyolefin-Material entfernt, indem es mittels eines Lösungsmittels aufgequollen wird, wobei sich die im Polyolefin-Material vorhandenen Verunreinigungen im Lösungsmittel lösen. Anschliessend werden das Lösungsmittel und die darin gelösten Verunreinigungen erst mechanisch vom Polyolefin-Material getrennt und anschliessend werden Lösungsmittelreste in einem Trockner entfernt.

In einem vierten Teil (dargestellt in Fig. 1d) wird das Polyolefin-Material nach Durchlaufen eines optionalen weiteren Sortierschritts geschmolzen, extrudiert, granuliert und einem SSP-Reaktor zugeführt, wo es einer Vakuum-Behandlung unterworfen wird.

Fig.1a: Das zu reinigende Polyolefin-Material stammt beispielsweise aus Behältern, insbesondere Flaschen, und Verschlüssen und kann in der Form von Ballen an die Recycling-Anlage angeliefert werden. Die Ballen werden in der Recycling-Anlage auseinander genommen (11). Die Behälter werden einem ersten Metallabscheider (12) zugeführt, der magnetisches Metall (12a) abtrennt. Anschliessend durchlaufen die Behälter eine Trenn-Stufe zur Abtrennung von Labels, d.h. einem "Label-Remover" (13), bevor sie einem Ballistik-Sorter (14) zugeführt werden, der Folien (14a) entfernt. In einem zweiten Metallabscheider (15) werden nicht-magnetische Metalle (15a) abgetrennt, wobei danach eine Sortierung nach Farben in einem (Farb)-Flaschen-Sorter (16) erfolgt, der Behälter mit anderer als der gewünschten Farbe (16a) aussortiert und in einer Mischfraktion (16b) sammelt. Schliesslich werden die sortierten Behälter in einer Mühle (18) zu Flakes geschnitten, welche in einem Windsichter (19) von verbliebenen Labels und Sleeves (19a) getrennt werden.

Fig.1b: Die Flakes werden mit Wasser und waschaktiven Substanzen wie z.B. einer Lauge gewaschen (21), in einer Trenn-Stufe nach Dichte getrennt (22), wobei Fremdpolymere wie PET, PC und/oder PVC (22a) aussortiert werden. Eine anschliessende Wäsche in heissem Wasser (23) dient der Entfernung von Papier und Kartonfasern (23a), die in Form eines Schlamms anfallen. Eine Nachwäsche kann bei vorgängiger Verwendung einer Lauge auch der Laugenneutralisation (24) dienen, wobei die Flakes nach Durchlaufen der Wasch-Stufen getrocknet und einem Windsichter (25) zugeführt werden. Mittels eines NIR-Spektrometers (26) können verbliebene Fremdkunststoffe (26a) identifiziert werden, was ein Aussortieren derselben ermöglicht. Die Flakes werden schliesslich abgefüllt (27) und geprüft (28). Bei nicht bestandener Prüfung werden die Flakes als Sperrware (28a) aussortiert. Bestehen die Flakes die Prüfung, werden sie weiterverarbeitet (29).

Fig.1c: Den Flakes wird ein Lösungsmittel (im vorliegenden Beispiel n-Heptan) zugegeben und es erfolgt ein erstes Mischen der Flakes mit dem Lösungsmittel durch Rühren (31). Unter erhöhtem Druck und bei erhöhter Temperatur nehmen die Flakes das Lösungsmittel auf, d.h. sie quellen (32). Nicht von den Flakes aufgenommenes Lösungsmittel wird durch einen Filter entfernt und die Flakes werden anschliessend ausgepresst (33), wodurch in den Flakes vorhandenes Lösungsmittel und darin gelöste Verunreinigungen (33a) aus den Flakes entfernt werden. Die vorgenannten Schritte werden ein (oder auch mehrere Male) wiederholt (34,35,36), um in den Flakes verbliebene Verunreinigungen (36a) zu entfernen. Die ausgepressten Flakes werden schliesslich getrocknet (37), wobei in und an den Flakes verbliebenes Lösungsmittel zusammen mit verbliebenen flüchtigen Verunreinigungen (37a) verdampft wird. Das Lösungsmittel und die Verunreinigungen können mittels einer Kältefalle getrennt werden, wobei die Verunreinigungen in einem Brenner verbrannt werden können (37). Die getrockneten Flakes werden wiederum geprüft (38). Nur solche Flakes, die die Prüfung bestehen, werden weiterverarbeitet (33), der Rest wird als Sperrware (38a) aussortiert.

Fig.1d: Die Flakes (41), welche in einem Silo (42) gesammelt wurden, werden optional einem (dritten) Metallabscheider (43) zugeführt, in dem Flakes aus Metall (43a) entfernt werden. Weiter werden die Flakes geschmolzen, extrudiert und granuliert (44), wobei die Extrusion eine Schmelzeentgasung (44a) umfassen kann, durch welche verbliebenes Lösungsmittel und/oder flüchtige Verunreinigungen entfernt werden können.

Das durch die Granulierung erzeugte Polyolefin-Granulat (44c) kann gesammelt und abgefüllt werden (44b). Dieses, gegebenenfalls noch nicht für die Verwendung im Lebensmittel-Bereich geeignete Granulat (44c) kann geprüft werden (44d), wobei es bei bestandener Prüfung zur Verwendung zur Verwendung für Lebensmittelverpackungen freigegeben (50) und bei nicht bestandener Prüfung aussortiert wird (Sperrware, 49a).
Alternativ kann das Pololefin-Material nach der Granulierung geprüft werden (45). Sollte die Farbe des Granulats nicht den Anforderungen entsprechen, können Additive zugegeben werden (45b), wobei das so modifizierte Polyolefin-Material zurück in das Silo (42) geführt werden kann. Besteht das Granulat die Prüfung (45) kann es direkt für Anwendungen ausserhalb des Lebensmittel-Bereichs zur Verfügung gestellt werden (45a) oder es wird einem SSP-Reaktor (46) zugeführt, in dem es einer Vakuum-Behandlung unterzogen wird. Das so behandelte Granulat wird gesammelt (47) und ist für die Verwendung im Lebensmittelbereich vorgesehen (48), wobei es vor der Freigabe (50) zu diesem Zweck geprüft wird (49). Bei bestandener Prüfung erfolgt die Freigabe (50), bei nicht bestandener Prüfung wird es als Sperrware aussortiert (49a).

## Patentansprüche

1. Recycling-Verfahren für verunreinigte Polyolefine, beinhaltend die Entfernung von Verunreinigungen aus einem verunreinigten Polyolefin-Material (11), wobei
- ein verunreinigtes Polyolefin-Material (11) mittels eines Lösungsmittels aufgequollen wird (31,34), um im Polyolefin-Material vorhandene Verunreinigungen zu entfernen, und
- das Lösungsmittel und die im Lösungsmittel gelösten Verunreinigungen aus dem Polyolefin-Material entfernt werden (33a,36a) und
- das verunreinigte Polyolefin-Material vor dem Aufquellen zu Stücken, zum Beispiel Flakes, verarbeitet worden ist und/oder es beim Aufquellen bereits in der Form von Stücken vorliegt,
**dadurch gekennzeichnet,**
**dass** die Stücke eine maximale Abmessung von höchstens 40, 30 oder 25 Millimeter aufweisen, wobei die maximale Abmessung durch den Abstand der am weitesten voneinander entfernten Punkte eines Stückes definiert ist.

2. Verfahren nach Anspruch 1, **dadurch gekennzeichnet, dass** die Stücke eine maximale Abmessung von mindestens 0.25, 0.5 oder 1 Millimeter aufweisen.

3. Verfahren nach Anspruch 1 oder 2, **dadurch gekennzeichnet, dass** das Polyolefin-Material im Wesentlichen aus Polyethylen oder Polypropylen besteht.

4. Verfahren nach einem der vorangehenden Ansprüche, **dadurch gekennzeichnet, dass** die Entfernung der Verunreinigungen aus dem Polyolefin-Material eine Kompression des aufgequollenen Polyolefin-Materials beinhaltet, wodurch ein Grossteil des Lösungsmittels aus dem aufgequollenen Polyolefin-Material gepresst wird, wobei das ausgepresste Polyolefin-Material vorzugsweise danach in einem Trockner getrocknet wird, wodurch im ausgepressten Polyolefin-Material verbleibendes Lösungsmittel verdampft wird.

5. Verfahren nach einem der vorangehenden Ansprüche, **dadurch gekennzeichnet, dass** das Polyolefin-Material geschmolzen und extrudiert wird, wobei dazu vorzugsweise ein Extruder mit Entgasungszone eingesetzt wird, um verbleibendes Lösungsmittel aus dem Polyolefin-Material zu entfernen, wobei das Polyolefin-Material im Anschluss an die Extrusion granuliert wird, wodurch ein Polyolefin-Granulat erhalten wird.

6. Verfahren nach Anspruch 5, **dadurch gekennzeichnet, dass** das Polyolefin-Granulat in einem Festphasenpolykondensationsreaktor (SSP-Reaktor) erhitzt wird, wobei dies vorzugsweise unter Vakuum erfolgt.

7. Verfahren nach einem der vorangehenden Ansprüche, **dadurch gekennzeichnet, dass** das vorzugsweise in der Form von Behältern vorliegende verunreinigte Polyolefin-Material, bevor es in der Gegenwart des Lösungsmittels aufgequollen wird, zu Flakes zerkleinert wird und Fremdmaterialien, wie zum Beispiel Papier oder Metall, durch einen oder mehrere Sortiervorgänge im trockenen Zustand und/oder durch ein oder mehrere Waschvorgänge in Wasser vom Polyolefin-Material getrennt werden.

8. Verfahren nach einem der vorangehenden Ansprüche, **dadurch gekennzeichnet, dass** das verunreinigte Polyolefin-Material bei einer Temperatur von etwa 10°C unterhalb der Schmelztemperatur des jeweiligen Polyolefinmaterials und einem Druck zwischen 1 und 1000 bar während mindestens 5 Minuten mittels des Lösungsmittels aufgequollen wird, wobei das Polyolefin-Material währenddessen bewegt, insbesondere gerührt wird.

9. Verfahren nach einem der vorangehenden Ansprüche, **dadurch gekennzeichnet, dass** das verunreinigte Polyolefin-Material mittels des Lösungsmittels aufgequollen wird, wobei das Volumen des verunreinigten Polyolefin-Materials durch das Aufquellen um mindestens 1 Prozent zunimmt.

10. Verfahren nach einem der vorangehenden Ansprüche, **dadurch gekennzeichnet, dass** es sich beim Lösungsmittel um ein Lösungsmittel handelt, welches weniger polar ist als Wasser ist, wobei es sich beim Lösungsmittel vorzugsweise um ein Alkan handelt, insbesondere um n-Hexan oder n-Heptan.
